# EUROPÄISCHE PATENTSCHRIFT

(11) **EP 3 280 382 B1**
(45) Veröffentlichungstag und Bekanntmachung des Hinweises auf die Patenterteilung: **24.06.2020**
(21) Anmeldenummer: 16716015.9
(22) Anmeldetag: 05.04.2016
(51) Int. Cl.: A61K 8/34, A61K 8/368, A61K 8/44, A61Q 19/00, A61Q 19/10

(54) **STABILE LÖSUNGEN VON CARBONSÄUREN UND CARBONSÄURESALZEN IN WÄSSRIGEN ALKANDIOLEN UND DEREN VERWENDUNG**
STABLE SOLUTIONS OF CARBOXYLIC ACIDS AND CARBOXYLIC ACID SALTS IN AQUEOUS ALKANEDIOLS AND THEIR USE
DES SOLUTIONS STABLES CONTENANT DES ACIDES CARBOXYLIQUES ET DES SELS D'ACIDES CARBOXYLIQUE ET LEUR UTILISATION

(30) Priorität: 09.04.2015 DE 102015105386
(43) Veröffentlichungstag der Anmeldung: 14.02.2018
(73) Patentinhaber: Minasolve SAS, 59310 Beuvry-la-Forêt (FR)
(72) Erfinder: KONATÉ, Nadia, 39108 Magdeburg (DE); NAHRWOLD, Markus, 32423 Minden (DE)
(74) Vertreter: Brantsandpatents bvba
(86) Internationale Anmeldenummer: PCT/EP2016/057407
(87) Internationale Veröffentlichungsnummer: WO 2016/162326

(56) Entgegenhaltungen:
- US-A1- 2004 096 528
- US-A1- 2007 258 936
- US-A1- 2008 139 672
- US-A1- 2012 201 902

## Beschreibung

Körperpflegemittel und Kosmetika bieten als wässrige Gemische einen guten Nährboden für Mikroorganismen. Erst eine ausreichende Konservierung dieser Produkte verhindert das Wachstum pathogener Keime, ermöglicht die Lagerung der Produkte über längere Zeiträume und gewährleistet die Sicherheit der Verbraucher. Zum Erreichen dieser Ziele werden üblicherweise Konservierungsmittel eingesetzt. Die hierzu am häufigsten verwendeten Substanzen können jedoch neben ihrer erwünschten schützenden Eigenschaft auch unerwünschte Nebenwirkungen zeigen. So wurde bei einigen der als "Parabene" bekannten 4-Hydroxybenzoesäureester eine schwache hormonelle Wirkung nachgewiesen. Isothiazolinon-Derivate erwiesen sich als sensibilisierend für signifikante Teile der Bevölkerung. Duftstoffe mit konservierenden Eigenschaften, wie Benzylalkohol oder Farnesol, wurden als allergieauslösend eingestuft. Die Verwendung Formaldehydabspaltender Substanzen wird aufgrund der bekannten krebserzeugenden Wirkung des Formaldehyds zunehmend als fragwürdig angesehen. Vor diesem Hintergrund ist die effektive und sichere Konservierung eine Herausforderung für alle Hersteller kosmetischer Produkte.

Ein weiteres Problem in Bezug auf die Konservierung stellt der anhaltende Trend zur Vermarktung zertifizierter "natürlicher" Kosmetik dar. Damit einher geht ein freiwilliger weitgehender Verzicht auf den Einsatz chemischer Konservierungsmittel, sowie die Verwendung von Rohstoffen natürlichen Ursprungs, die vielfach gute Nährstoffe für Mikroorganismen darstellen. Zur Konservierung werden vermehrt auch botanische Extrakte mit antimikrobieller Wirkung verwendet, deren Zusammensetzungen jedoch variieren können und deren Inhaltsstoffe nicht immer vollständig charakterisiert sind.

Weiterhin sind viele der aktuell in Kosmetika eingesetzten Konservierungsmittel fettlösliche Substanzen. Dementsprechend begrenzt ist ihre effektive Konzentration in wässrigen Lösungen. In mehrphasigen Gemischen besteht zudem die Möglichkeit, dass die fettlöslichen Konservierungsstoffe von der Wasserphase in die Ölphase übergehen. Mikroorganismen vermehren sich jedoch bevorzugt in einem wässrigen Milieu oder an Grenzflächen zu wässrigen Phasen. Eine verminderte Verfügbarkeit der Konservierungsmittel in der wässrigen Phase reduziert daher ihre antimikrobielle Wirksamkeit.

Eine Möglichkeit zur effektiven Konservierung von Kosmetika besteht in der Verwendung bestimmter Carbonsäuren, wie z.B. Sorbinsäure, Benzoesäure oder Salicylsäure. Diese Carbonsäuren sind weltweit als Konservierungsmittel für Kosmetika zugelassen, unter anderem auch in der EU-Kosmetikverordnung. Weiterhin sind die genannten Substanzen konform mit Richtlinien für Naturkosmetik, beispielsweise mit dem internationalen Standard "COSMOS". Nachteilig ist jedoch die oftmals geringe Wasserlöslichkeit der freien Carbonsäuren, die zudem als Feststoffe schwer handhabbar sein können. Weiterhin wird durch Zugabe freier Carbonsäuren der pH-Wert entsprechender Formulierungen deutlich abgesenkt, was zu Stabilitätsproblemen führen und eine nachfolgende Neutralisation erforderlich machen kann. Zwar besteht die Möglichkeit, die Carbonsäuren in Form ihrer wasserlöslichen Alkalimetall- oder Ammoniumsalze einzusetzen. Allerdings sind Carbonsäuren insbesondere in freier Form antimikrobiell aktiv, während die deprotonierte Form deutlich schwächer konservierend wirkt. Zur Protonierung der konservierenden Säuren ist daher ein nachfolgendes Ansäuern der Produkte erforderlich. Dadurch werden zusätzliche potentiell hautirritierende Säuren in die Formulierung eingebracht und die Zusammensetzung der Formulierung wird weiter verkompliziert.

Eine weitere Möglichkeit zum Schutz von Kosmetika vor mikrobiologischer Kontamination stellt der Einsatz von neutralen Polyolen mit antimikrobieller Wirkung dar. Dabei finden vor allem Alkandiole Verwendung. Insbesondere 1,2-Octandiol und 1,2-Decandiol zeigen eine eigenständige konservierende Wirkung. Aufgrund ihrer hautirritierenden Wirkung sind sie jedoch nur in begrenzter Konzentration einsetzbar. Außerdem sind diese beiden Substanzen lipophil und kaum wasserlöslich. Somit besteht eine hohe Wahrscheinlichkeit, dass diese beiden Substanzen nur begrenzt in wässrigen Phasen verfügbar sind. Darüber hinaus sind 1,2-Octandiol und 1,2-Decandiol nicht geruchlos. Sie zeigen weiterhin eine starke Tendenz zur Destabilisierung von O/W-Emulsionen. Demgegenüber weisen kürzerkettigen Diole wie z.B. 1,2-Pentandiol und 1,2-Hexandiol eine erhöhte Hautkompatibilität auf. Sie sind geruchlos, wasserlöslich und weitgehend kompatibel zu O/W-Emulsionen und anderen galenischen Formen. Allerdings ist ihre antimikrobielle Aktivität deutlich geringer ausgeprägt, als bei den längerkettigen Diolen. Für eine kosteneffektive Konservierung kosmetischer Produkte werden die kürzerkettigen Diole daher in der Regel mit anderen Konservierungsmitteln kombiniert. Durch synergistische Effekte kann dabei die Gesamtmenge an Konservierungsmitteln geringer ausfallen, als es durch Addition der einzelnen antimikrobiellen Effekte zu erwarten gewesen wäre ("preservative boosting").

In der Patentliteratur werden einige Kombinationen aus Alkandiolen und Carbonsäuren mit vorteilhafter antimikrobieller Wirkung beschrieben. Diese weisen jedoch jeweils auch Nachteile auf:
EP1598064A1 offenbart eine synergistische antimikrobielle Wirkung von alpha- und beta-Hydroxycarbonsäuren in Kombination mit 1,2-Alkandiolen gegen das Bakterium *Propionibacterium acnes.* Als bevorzugtes Beispiel wird die Kombination von Milchsäure mit 1,2-Decandiol beschrieben. In den aufgeführten Anwendungsbeispielen werden Alkandiole jedoch nicht als Lösemittel eingesetzt, sondern in geringeren Konzentrationen als die Carbonsäuren. Somit weisen die offenbarten Mischungen einen niedrigem pH-Wert auf. Die Eignung mittelkettiger 1,2-Alkandiole als gleichzeitig effektives Lösemittel für Carbonsäuren und Carbonsäuresalze, sowie die Stabilität und die vorteilhafte Anwendung von Lösungen bestehend aus Alkandiolen, Carbonsäuren und Carbonsäuresalzen wird nicht erwähnt. Ebenfalls nicht erwähnt wird die allgemeine konservierende Wirkung derartiger Mischungen.
US2008139672A1 offenbart die synergistischen antimikrobiellen Effekte fettlöslicher Glykole mit weiteren Konservierungsmitteln. Dabei werden 1,2-Pentandiol, sowie verschiedene Carbonsäuren und Carbonsäuresalze explizit als Komponenten genannt. Eine vereinfachte Anwendung derartiger Mischungen in Bezug auf den pH-Wert und die allgemeine Formulierung kosmetischer Zubereitungen wird erwähnt. Auf die konkrete Zusammensetzung entsprechender lagerstabiler Konzentrate, ggf. unter Zusatz geringer Mengen Wasser, wird jedoch nicht eingegangen. Nicht erwähnt wird weiterhin die mögliche Mischbarkeit derartiger Lösungen mit Wasser, sowie deren Lagerstabilität bei tiefen Temperaturen. Die aufgeführten Beispiele erwähnen als Diol-Komponente lediglich 1,2-Octandiol, welches kaum wasserlöslich ist und bei tiefen Temperaturen erstarrt. Die Eignung auch kürzerkettiger Diole als Konservierungsmittel ist auf Grundlage der aufgeführten Daten nicht offensichtlich. Weiterhin unerwähnt bleibt die Möglichkeit, den pH-Wert einer Carbonsäure-Lösung auf Diol-Basis durch gezielten Zusatz bestimmter Mengen an Carbonsäuresalzen einzustellen, wodurch eine gute Verfügbarkeit der Konservierungsmittel in wässriger Lösung gewährleistet werden kann.
KR102011139527A offenbart die kombinierte Anwendung von Carbonsäuren und hautbefeuchtenden Substanzen zur Herstellung selbstkonservierender Hautpflegemittel. Die Herstellung entsprechender stabiler Lösungen dieser Komponenten wird nicht beschrieben, und auch die Anwendung von Carbonsäuresalzen zur pH-Werteinstellung wird nicht erwähnt. In konkreten Anwendungsbeispielen werden ausschließlich 1,2-Octandiol und Glycerylmonocaprylat mit verschiedenen Carbonsäuren kombiniert. Die Eignung kürzerkettiger Diole als Konservierungsmittel ist aus den aufgeführten Daten nicht ersichtlich. Ebenfalls nicht erwähnt wird die vorteilhafte gleichzeitige Kombination von Carbonsäuren und Carbonsäuresalzen mit Diolen.
WO2009099419A2 offenbart die Anwendung verdünnter wässriger Lösungen von Säuren und Diolen als antimikrobielle Zubereitungen für Haushaltsprodukte, landwirtschaftliche Produkte und medizinische Anwendungen. In konkreten Anwendungsbeispielen wird Wasser als hauptsächliches Lösemittel für die antimikrobiellen Lösungen verwendet. Als Diol wird 1,2-Propandiol in einer Konzentration von maximal 3 % eingesetzt. Die Konzentration der organischen Säuren im Konzentrat ist zum Teil deutlich höher als die des Diols. Dies kann mutmaßlich zu Hautirritationen bei der Anwendung in Kosmetika führen. Die anti-mikrobielle Wirkung der beschriebenen wässrigen Lösungen wird hauptsächlich durch deren niedrigen pH-Wert erzielt, was im Zusammenhang mit kosmetischen Formulierungen wenig praktikabel ist. Durch die starke Verdünnung mit dem inaktiven Lösemittel Wasser müssen die Lösungen in entsprechend größerer Menge eingesetzt werden, was den Spielraum zum Einsatz weiterer kosmetischer Rohstoffe einengt.
US20060229291A1 offenbart wässrige Lösungen von Mischungen aus p-Methoxybenzoesäure (Anissäure) und dessen Salz Natriumanisat, die durch Zusatz mehrwertiger Alkohole (Polyole) stabilisiert werden. Die als Anwendungsbeispiele aufgeführten Lösungen enthalten jedoch > 25 % Wasser als physiologisch inaktives Lösemittel, welches lediglich der physikalischen Stabilisierung dient. Für die Neutralisation der p-Methoxybenzoesäure wird wässrige Natronlauge verwendet. Zur Erzielung neutraler Lösungen ist hierbei eine genaue Titration notwendig. Nicht beschrieben wird die Neutralisation von Carbonsäuren durch Vermischung mit festen Carboxylat-Salzen. Diese Art der Neutralisation würde durch Einwaage erfolgen, wodurch eine aufwändige Titration vermieden würde. Als Polyole werden insbesondere Glycerin und dessen Derivate eingesetzt, die auf Grund ihrer Hydrophilie lediglich ein eingeschränktes Lösevermögen gegenüber lipophilen Carbonsäuren aufweisen. Darüber hinaus weisen diese Diole nur eine vergleichsweise geringe eigenständige antimikrobielle Wirkung auf.
WO2006045743A1 offenbart synergistische antimikrobielle Mischungen bestehend aus 1,2-Hexandiol, 1,2-Octandiol, sowie optional aus 1,2-Pentandiol und 1,2-Decandiol. Die Lösungen können ferner optional das Carbonsäuresalz Kaliumsorbat enthalten. In jedem Fall werden die fettlöslichen Alkandiole 1,2-Octandiol und 1,2-Decandiol eingesetzt, mit den sich daraus ergebenden Nachteilen bezüglich Geruchs, geringer Wasserlöslichkeit und möglicher Hautirritation. Über die Stabilität entsprechender Lösungen bestehend aus den genannten Komponenten wird nicht berichtet.
US020080311231A1 offenbart Mischungen enthaltend und/oder bestehend aus den lipophilen Diolen 1,2-Octandiol und/oder Ethylhexylglycerin und alpha-Hydroxycarbonsäuren, optional unter Zusatz von Wasser oder 1,2-Hexandiol. Die Verwendung von Metallsalzen der Carbonsäuren wird explizit ausgeschlossen. Die entsprechenden Lösungen erzeugen allesamt einen deutlichen pH-Wertabfall bei Zugabe zu kosmetischen Produkten, mit den daraus resultierenden Nachteilen. Außerdem sind die beschriebenen Lösungen nur stark begrenzt mit Wasser mischbar, weshalb sie nicht uneingeschränkt für wasserbasierte Systeme einsetzbar sind.

### Zielsetzung der Erfindung

In Anbetracht des oben beschriebenen Standes der Technik besteht ein Bedarf an Konservierungsmitteln für Kosmetika und dermatologische Produkte, die folgende Eigenschaften aufweisen:
- Breitband-Konservierungseffekt in Kosmetika gegen Bakterien, Pilzen und Hefen
- wasserlöslich bis mindestens zur höchsten in Kosmetika zugelassenen Konzentration
- minimaler Anteil inaktiver Hilfsstoffe
- kompatibel zu gängigen kosmetischen und dermatologischen Produkten
- geringer Einfluss auf den pH-Wert gängiger kosmetischer Produkte
- zu beliebigen Zeitpunkten innerhalb der Produktion von Fertigprodukten einsetzbar
- im kalten Zustand verwendbar ("cold processing" kompatibel)
- homogene, physikalisch und chemisch stabile Flüssigkeit
- weitgehend geruchlos und farblos
- wenn möglich konform zu Standards und Richtlinien für Naturkosmetika.

### Detaillierte Beschreibung der Erfindung

Die Aufgabe wurde überraschend gelöst durch eine Lösung, die insbesondere zur Verwendung zur Konservierung kosmetischer und dermatologischer Produkte geeignet ist, wobei die Lösung aus den Komponenten a) bis d) besteht:
a) zu 40 - 95 Gew.-% mindestens eines C₅-C₆Alkandiols,
b) zu 0,1 - 35 Gew.-% mindestens einer Carbonsäure aus der Gruppe Benzoesäure, Salicylsäure und Capryloylglycin,
c) zu 0,1 - 35 Gew.-% mindestens eines Salzes einer C₆-C₉-Carbonsäure,
d) zu 0,1 - 25 Gew.-% Wasser,
wobei sich die Anteile a, b, c und d zu 100 Gew.-% addieren.

In anderen Worten werden Carbonsäuren und Carbonsäuresalze gemeinsam in einem Alkandiol gelöst, das wasserlöslich ist und einen log *K*_{ow}-Wert von -1,0 bis +1,0 aufweist. Dabei wurde gefunden, dass derartige Alkandiole gute Lösemittel sowohl für lipophile Carbonsäuren, als auch für hydrophile Carbonsäuresalze sind. Auch bei maximaler Nutzung der oben genannten Gew.-%-Angaben für die einzelnen Komponenten, z.B. 40 Gew.-% Alkandiol in Verbindung mit bis zu 35 Gew.-% Carbonsäure oder bis zu 35 Gew.-% Carbonsäuresalz liegt in bestimmten Fällen immer noch eine Lösung vor, d.h. das Alkandiol zusammen mit Wasser dient als Lösungsmittel für Carbonsäure und -salz (siehe Beispiel 4D).

Die erfindungsgemäßen Lösungen sind in einem weiten Temperaturbereich klar, stabil und homogen. Überraschenderweise sind Lösungen, die gleichzeitig Carbonsäuren und deren Salze enthalten, stabiler als Lösungen, die ausschließlich reine Carbonsäuren oder reine Carbonsäuresalze enthalten. Eine Auskristallisation der festen Komponenten bei niedriger Temperatur wird dadurch vermieden. Weiterhin unerwartet kann die Stabilität der Lösungen nochmals erhöht werden, wenn maximal 25 Gew.-% Wasser zu der Mischung zugesetzt werden. Der pH-Wert der erfindungsgemäßen Lösungen liegt zwischen 4 und 8.

Kälte- und wärmestabile flüssige Mischungen wie die erfindungsgemäßen Lösungen sind vorteilhaft, da sie auch bei Lagerung in kalter oder warmer Umgebung ihre Zusammensetzung beibehalten. Sie müssen daher während eines Transports nicht beheizt oder gekühlt werden. Auch unmittelbar vor ihrer Anwendung müssen die Lösungen nicht homogenisiert werden, wodurch Zeit, Energie und Produktionskapazität eingespart werden. Flüssige Mischungen erlauben generell eine einfache und genaue Dosierung, z.B. durch Zupumpen, Eingießen oder Einsaugen. Die Einarbeitung in flüssige Produkte ist schneller, effektiver und gleichmäßiger als die Einarbeitung als Feststoff. Beispielsweise wird eine Klumpenbildung vermieden. Flüssige Konzentrate ermöglichen außerdem durch Vermeidung von Stäuben eine sicherere Handhabung von ansonsten haut-, schleimhaut- oder augenreizenden Substanzen.

Überraschenderweise bleibt die antimikrobielle Aktivität der Lösungen vollständig erhalten, wenn neutrale Carbonsäuren teilweise durch ihre theoretisch weniger antimikrobiell aktiven Carbonsäuresalze ersetzt werden. Eine mögliche Erklärung ist die in beiden Fällen vergleichbar hohe Verfügbarkeit der Carbonsäuren in der wässrigen Phase der konservierten Produkte.

Nicht vorhersehbar war weiterhin, dass die eingesetzten wasserlöslichen und kurzkettigen Alkandiole in Kosmetika bereits in geringen Einsatz-Konzentrationen von ≤ 2 Gew.- % als Breitband-Konservierungsmittel wirken, wenn sie zusammen mit insgesamt ≤ 0,2 Gew.- % Carbonsäure(n)- und/oder Carbonsäuresalz(en) - eingesetzt werden (Gew.-%-Angaben hier bezogen auf die Gesamtmenge inklusive Kosmetikum). Keine der einzelnen Komponenten allein wäre in den genannten Konzentrationen dazu in der Lage, die entsprechenden Produkte effektiv zu konservieren. Dabei sind die erfindungsgemäßen Lösungen in der Lage, diverse Mikroorganismen abzutöten. Generell umfasst der Ausdruck "Mikroorganismus" Bakterien und Pilze, insbesondere gram-positive und gram-negative Bakterien, sowie Hefen und Schimmelpilze. Beispiele für Zielorganismen (ohne ausschließenden Charakter) sind: *Escherichia coli, Staphylococcus aureus, Enterococcus hirae, Pseudomonas aeruginosa, Burkholderia cepacia, Candida albicans* und/oder *Aspergillus brasiliensis.*

Als wasserlösliche Alkandiol-Komponente wird ein C5-C6-Alkandiol verwendet, besonders bevorzugt ein vicinales C5-C6-Alkandiol. Besonders bevorzugt sind 1,2-Pentandiol und 1,2-Hexandiol. Insbesondere bevorzugt ist 1,2-Pentandiol, das aus pflanzlichen Rohstoffen hergestellt wurde und somit den Maßstäben für naturkosmetische Rohstoffe entspricht. Die Einsatzkonzentration des mindestens einen Alkandiols (a) liegt bei 40 - 95 Gew. "Mindestens ein Alkandiol" bedeutet einzelne Alkandiole (a), aber auch Mischungen aus mehreren, voneinander unterschiedlichen Alkandiolen (a). Bevorzugt wird ein einzelnes Alkandiol (a) eingesetzt.

Das im Produkt enthaltene mindestens eine Alkandiol kann zusätzliche Funktionen innerhalb des Fertigproduktes haben, z.B. als Feuchthaltemittel, als penetrationsförderndes Reagenz oder als Mittel zur Beeinflussung des Hautgefühls.

Als mindestens eine Carbonsäure (b) wird die mindestens eine C6-C9-Carbonsäure aus der Gruppe Benzoesäure, Salicylsäure und Capryloylglycin eingesetzt, die für kosmetische Anwendungen geeignet ist. Die Einsatzkonzentration der mindestens einen Carbonsäure liegt bei 0,1 - 35 Gew.-%. "Mindes-tens eine Carbonsäure bedeutet, dass einzelne Carbonsäuren, aber auch Mischungen mehre-rer verschiedener Carbonsäuren umfasst sind.

Als mindestens ein Carbonsäuresalz (c) werden Carboxylate einer C6-C9-Carbonsäure eingesetzt, insbesondere solche, die für einen Einsatz in kosmetischen Produkten geeignet sind. "Mindestens ein Carbonsäuresalz" bedeutet, dass einzelne Carbonsäuresalze, aber auch Mischungen mehrerer verschiedener Carbonsäuresalze umfasst sind.

Vorzugsweise werden Salze der oben aufgeführten Carbonsäuren eingesetzt, die für einen Einsatz in kosmetischen Produkten geeignet sind. In anderen Worten wird als das mindestens eine Carbonsäuresalz (c) vorzugsweise ein Carboxylat der Carbonsäure (b) eingesetzt.

Das Gegenion des Carboxylats im Carbonsäuresalz (c) ist ausgewählt aus Ammonium-, Triethanolammonium-, Natrium-, Kalium-, Calcium-, und Magnesiumsalze. Besonders bevorzugt werden Natrium- und Kaliumsalze verwendet. Die Einsatzkonzentration des mindestens einen Carbonsäuresalzes liegt bei 0,1 - 35 Gew.-%.

Carbonsäure und korrespondierendes Carboxylat bzw. Carbonsäuresalz werden vorzugsweise in einem molaren Verhältnis zueinander von 1:10 bis 10:1, mehr bevorzugt von 1:5 bis 5:1, noch mehr bevorzugt von 1:2 bis 2:1 eingesetzt.

Zur Stabilisierung der erfindungsgemäßen Lösungen wird Wasser in einer Konzentration von 0,1 - 25 Gew.-% eingesetzt, bevorzugt in einer Konzentration von 5 - 20 Gew.-%. Unabhängig von ihren konservierenden und pH-regulierenden Eigenschaften können die in den erfindungsgemäßen Lösungen enthaltenen Carbonsäuren und Carbonsäuresalze auch andere Aufgaben erfüllen. Beispiele ohne ausschließenden Charakter sind Anwendungen als Feuchthaltemittel, Konditionierer, Hautaufheller, Peeling-Mittel, Enzyminhibitor, Antioxidans, Licht- und UV-Schutz, zur Regulierung der Talgproduktion, zur Beeinflussung der Rheologie bzw. der Viskosität, sowie zur positiven Beeinflussung altersbedingter oder umweltbedingter Hautveränderungen.

Ebenso erfindungsgemäß ist ein Produkt, insbesondere ein kosmetisches und/oder pharmazeutisches und/oder dermatologisches und/oder hygienisches Produkt, enthaltend eine Lösung wie oben erläutert. In anderen Worten wird die erfindungsgemäße Lösung zur Konservierung kosmetischer und/oder pharmazeutisches und/oder dermatologischer und/oder hygienischer Produkte, insbesondere kosmetischer und dermatologischer Produkte, insbesondere zur Wachstumshemmung oder Abtötung von Mikroorganismen verwendet. Als "hygienische Zubereitung" bzw. "hygienisches Produkt" werden insbesondere Haushalts- oder Reinigungsprodukte, sowie Riechstoffzubereitungen verstanden.

Die Zugabe der erfindungsgemäßen Lösungen zu dem kosmetischen und/oder pharmazeutischen und/oder dermatologischen und/oder hygienischen Produkt kann zu jedem beliebigen Zeitpunkt während der Produktion erfolgen, z.B. während der Herstellung einer wässrigen Phase oder am Ende des Produktionsablaufs.

Im Umfang der Erfindung ist ebenfalls ein Verfahren zur Wachstumshemmung oder Abtötung von Mikroorganismen enthalten, bei welchem die erfindungsgemäßen Lösungen zu Produkten aus den Anwendungsbereichen Kosmetik, Dermatologie, Körperpflege und Körperhygiene zugesetzt werden.

Das jeweilige Produkt kann in beliebiger Form vorliegen, insbesondere als:
a. Lösung,
b. Suspension,
c. Emulsion,
d. Gel,
e. Salbe,
f. Paste,
g. Pulver,
h. in Stücken oder als Block vorliegender Feststoff,
i. Schaum,
j. auf Mikroverkapselung, Liposomen oder ähnlichen mikroskopischen Strukturen basierendes Formulierungssystem.
k. Kombinationen der Formen a - j

### Beispiele / experimenteller Teil

### Beispiel 1 - Herstellung erfindungsgemäßer Lösungen

a) 1,2-Pentandiol (153 g) wird vorgelegt. Benzoesäure (7 g) und Natriumbenzoat (13 g) werden zugegeben, gefolgt von Wasser (27 g). Die Mischung wird so lange gerührt, bis eine klare Lösung entstanden ist. Der pH-Wert der Lösung beträgt 6,0.
b) 1,2-Pentandiol (136 g) wird vorgelegt. Capryloylglycin (30 g) und Natriumsalicylat (100 g) werden zugegeben, gefolgt von Wasser (34 g). Die Mischung wird so lange gerührt, bis eine klare Lösung entstanden ist. Der pH-Wert der Lösung beträgt 5,0.
c) 1,2-Pentandiol (204 g) wird vorgelegt. Salicylsäure (21 g) und Natriumsalicylat (45 g) werden zugegeben, gefolgt von Wasser (30 g). Die Mischung wird so lange gerührt, bis eine klare Lösung entstanden ist. Der pH-Wert der Lösung beträgt 4,5.

### Beispiel 2 - pH-Wert-Einstellung mit Hilfe des Carbonsäure/Carbonsäuresalz-Verhältnisses:

Zur Einstellung des pH-Wertes der erfindungsgemäßen Lösungen können Carbonsäure und Carbonsäuresalz in unterschiedlichen Mengenverhältnissen eingesetzt werden, wie nachfolgend am Beispiel von Benzoesäure und Natriumbenzoat gezeigt:

| **Lösung** | **1,2-Pentandiol** | **Wasser** | **Natriumbenzoat** | **Benzoesäure** | **pH** |
|---|---|---|---|---|---|
| **A** | 76.5 g | 13.5 g | 9.6 g | 0.4 g | 7.23 |
| **B** | 76.5 g | 13.5 g | 9.5 g | 0.5 g | 6.94 |
| **C** | 76.5 g | 13.5 g | 9.0 g | 1.0 g | 6.63 |
| **D** | 76.5 g | 13.5 g | 8.0 g | 2.0 g | 6.25 |
| **E** | 76.5 g | 13.5 g | 6.5 g | 3.5 g | 5.90 |
| **F** | 76.5 g | 13.5 g | 5.0 g | 5.0 g | 5.61 |
| **G** | 76.5 g | 13.5 g | 3.0 g | 7.0 g | 5.31 |

Die Lösungen A-G sind klar und farblos bei 20 °C, unabhängig von dem jeweils eingestellten pH-Wert. Die Lösungen B (pH 6.94) und G (pH 5.31) wurden auf ihre Langzeitstabilität hin untersucht. Beide Lösungen erwiesen sich als lagerstabil bei 42 °C, 20 °C und 8 °C über einen Zeitraum von 3 Monaten.

### Beispiel 3 - Löslichkeit von Salzen in 1,2-Pentandiol mit/ohne Wasser

Um den positiven Einfluss einer geringen Wassermenge (≤ 25 %) auf die Löslichkeit von Carbonsäuren und Carbonsäuresalzen in Alkandiolen zu veranschaulichen, wurde exemplarisch die Löslichkeit von Natriumbenzoat in 1,2-Pentandiol nach Zusatz unterschiedlicher Mengen Wasser ermittelt. Dazu wurden jeweils gesättigte Lösung in den unterschiedlichen Lösemittelgemischen hergestellt. Nach Lagerung über Nacht bei 20-25 °C wurde der Feststoff jeweils abzentrifugiert. Der Massengehalt von Natriumbenzoat in den klaren flüssigen Überständen wurde mittels HPLC/UV ermittelt:

| **Lösung** | **A** | **B** | **C** | **D** | **E** |
|---|---|---|---|---|---|
| **1,2-Pentandiol** | 100 % | 95 % | 90 % | 85 % | 80 % |
| **Wasser** | 0 % | 5 % | 10 % | 15 % | 20 % |
| **Sättigungskonzentration Natriumbenzoat bei 20 °C** | 11.7 % | 12.4 % | 13.7 % | 15.7 % | 12.6 % |

Es zeigte sich, dass die Löslichkeit von Natriumbenzoat in 1,2-Pentandiol/Wasser-Gemischen ein Maximum bei einem Wassergehalt von ca. 15 % erreicht.

### Beispiel 4 - Temperaturstabilität erfindungsgemäßer Lösungen

| **Nr .** | **Alkandiol** | **Carbonsäure** | **Carbonsäure-salz** | **H₂O** | **pH** | **Kristallisationspunkt** | **Schmelzpunkt** |
|---|---|---|---|---|---|---|---|
| **A** | 1,2-Pentandiol (76,5 %) | Benzoesäure (5 %) | Natriumbenzoat (5 %) | 13,5 % | 5,7 | < -20 °C | < -20 °C |
| **B** | 1,2-Pentandiol (76,5 %) | Benzoesäure (3 %) | Natriumbenzoat (7 %) | 13,5 % | 6,0 | < -20 °C | < -20 °C |
| **C** | 1,2-Pentandiol (76,5 %) | Benzoesäure (1,5 %) | Natriumbenzoat (8,5 %) | 13,5 % | 6,4 | < -20 °C | < -20 °C |
| **D** | 1,2-Pentandiol (45,3 %) | Capryloylglycin (13,3%) | Natriumsalicylat (33,3 %) | 11,3 % | 5,0 | < -20 °C | < -20 °C |
| **E** | 1,2-Pentandiol (48,0 %) | Capryloylglycin (13,3%) | Natriumsalicylat (26,7 %) | 12,0 % | 5,0 | < -20 °C | < -20 °C |
| **F** | 1,2-Pentandiol (62,4 %) | Salicylsäure (8,8 %) | Natriumsalicylat (18,8 %) | 10,0 % | 4,5 | < -20 °C | < -20 °C |
| **G** | 1,2-Pentandiol (62,9 %) | Salicylsäure (7 %) | Natriumsalicylat (15,1 %) | 15,0 % | 4,5 | < -20 °C | < -20 °C |
| **H** | 1,2-Pentandiol (67,9 %) | Salicylsäure (7 %) | Natriumsalicylat (15,1 %) | 10,0 % | 4,5 | < -20 °C | < -20 °C |

Die Lösungen wurden während 16-20 h auf -20 °C temperiert. Anschließend wurden sie innerhalb von 10 h in 2 °C-Schritten auf +20 °C erwärmt. Die niedrigste Temperatur, bei der eine klare homogene Lösung vorlag, wurde als Schmelzpunkt betrachtet. Anschließend wurden die Mischungen innerhalb von 10 h in 2 °C-Schritten wieder auf -20 °C abgekühlt. Die höchste Temperatur, bei der eine heterogene Mischung mit Feststoffanteil vorliegt, wurde als Kristallisationspunkt betrachtet.

### Beispiel 5 - Wasserlöslichkeit erfindungsgemäßer Lösungen

Jeweils 1 g der in Beispiel 4A-H aufgeführten Lösungen wurde bei 20-25 °C in jeweils 19 g entionisiertes Wasser gegossen und im Reagenzglas vermischt. Dabei wurden jeweils klare wässrige Lösungen erhalten.

### Beispiel 6 - Konservierung einer O/W-Creme

| **Phase** | **Inhaltstoff** | **INCI-Name** | **%** |
|---|---|---|---|
| A | Wasser | *Water* | 69.5 |
| | Xanthan-Gummi | *Xanthan gum* | 0.3 |
| B | Emulium kappa 2 | *Candellila*/*jojoba*/*rice bran polyglyceryl-3 esters (and) Glyceryl stearate (and) Cetearyl alcohol (and) Sodium stearoyl lactylate* | 5.0 |
| | Glycerin-Monostearat | *Glyceryl monostearate* | 2.3 |
| | Tego Care PS | *Methyl glucose sesquistearate* | 1.2 |
| | Jojobaöl | *Simmondsia chinensis oil* | 5.0 |
| | Mandelöl | *Prunus amygdalus dulcis oil* | 4.0 |
| | Lipex Cocoasoft | *Theobroma cacao butter* | 3.0 |
| | BergaCare FG5 | *Ethylhexyl palmitate (and) Ethylhexyl stearate (and) Hydrogenated olive oil* | 2.0 |
| | Traubenkernöl | *Vitis vinifera seed oil* | 4.0 |
| | Bienenwachs | *Cera alba* | 1.5 |
| C | **Erfindungsgemäße Lösung gemäß Beispiel 4B** | *Pentylene glycol (and) Water (and) Sodium benzoate (and) Benzoic Acid* | 2.0 |
| | Parfüm | *Perfume* | 0.1 |
| | Tocopherol | *Tocopherol* | 0.1 |

Xanthan-Gummi wird in Wasser aufgenommen. Die Mischung wird unter Rühren auf 75-80 °C erhitzt. Phase B wird auf 75-80 °C erhitzt und unter Rühren zu Phase A gegeben. Die Mischung wird 10 min bei 2000 U/min gerührt und anschließend unter langsamerem Rühren abgekühlt. Bei 40 °C werden die Komponenten der Phase C nacheinander unter Rühren in der beschriebenen Reihenfolge zugesetzt. Der pH-Wert der erhaltenen Creme beträgt 5-6.

Das Produkt wurde einem Konservierungsmittelbelastungstest gemäß ISO 11930 unterzogen. Die Prüfung besteht aus der Kontamination des Prüfproduktes mit einem durch die Norm vorgeschriebenen Inokulum von fünf unterschiedlichen Arten von Mikroorganismen, der Entnahme von Proben aus dem Prüfprodukt nach 7, 14 und 28 Tagen und der Bestimmung der Anzahl von Testkeimen in den entnommenen Proben. Die konservierenden Eigenschaften sind ausreichend, wenn sich unter den Bedingungen der Prüfung eine eindeutige Verminderung oder ggf. keine Vermehrung der Keimzahl in dem beimpften Prüfprodukt nach den vorgeschriebenen Zeiten ergibt. Zur Beurteilung der konservierenden Wirkung der erfindungsgemäßen Lösung wurde das Produkt zum Vergleich auch ohne Zusatz der erfindungsgemäßen Lösung einem Konservierungsmittelbelastungstest unterzogen. Die Änderungen der Keimbelastung beider Prüfprodukte sowie die durch die Norm vorgegebenen Sollwerte sind in der nachfolgenden Tabelle protokolliert. Die angegebenen Werte entsprechen jeweils dem dekadischen Logarithmus der Anzahl der koloniebildenden Einheiten (KBE). Positive Zahlen repräsentieren eine Reduktion der Keimzahl, negative Zahlen einen Anstieg.

| | **Creme nach Beispiel 6, mit 2 % der Lösung 4B konserviert (Δ log KBE)** | | | **Creme nach Beispiel 6, jedoch ohne Lösung 4B (Δ log KBE)** | | | **ISO 11930, Kriterien A für ausreichende Konservierung (Δ log KBE)** | | |
|---|---|---|---|---|---|---|---|---|---|
| **Testkeim** | **T7** | **T14** | **T28** | **T7** | **T14** | **T28** | **T7** | **T14** | **T28** |
| **Aspergillus brasiliensis** | 1,1 | 1,0 | 1,4 | 0,4 | 1,2 | 1,2 | - | ≥ 0 | ≥ 1 |
| **Candida albicans** | 3,1 | > 3,4 | >3,4 | -0,4 | -0,8 | -0,8 | ≥ 1 | ≥ 1 | ≥ 1 |
| **Pseudomonas aeruginosa** | >4,8 | >4,8 | >4,8 | -0,2 | -0,9 | -0,9 | ≥ 3 | ≥ 3 | ≥ 3 |
| **Staphylococcus areus** | >4,6 | >4,6 | >4,6 | 0,8 | 0,7 | 0,7 | ≥ 3 | ≥ 3 | ≥ 3 |
| **Escherichia coli** | >4,4 | >4,4 | >4,4 | -0,5 | -1,2 | -1,2 | ≥ 3 | ≥ 3 | ≥ 3 |

Die mit 2 % der erfindungsgemäßen Lösung nach Beispiel 4B konservierte Creme erfüllt die A-Kriterien für ausreichende Konservierung kosmetischer Produkte gemäß ISO 11930, während die unkonservierte Creme die Kriterien der Norm für 4 der 5 Testkeime nicht erfüllt.

### Beispiel 7 - Konservierung einer O/W-Creme

| **Phase** | **Inhaltstoff** | **INCI-Name** | **%** |
|---|---|---|---|
| A | Wasser | *Water* | 82,4 |
| | Xanthan Gummi | *Xanthan gum* | 0,5 |
| B | Emulgade PL 68/50 | *Cetearyl Glucoside (and) Cetearyl Alcohol* | 5,0 |
| | Lipex Shea | *Butyrospermum Parkii (Shea) Butter* | 3,0 |
| | Jojobaöl | *Simmondsia Chinensis (Jojoba) Seed Oil* | 3,0 |
| | Haselnussöl | *Corylus A vellana (hazelnut) oil* | 3,0 |
| C | **Erfindungsgemäße Lösung gemäß Beispiel 4E** | *Pentylene glycol (and) Sodium salicylate (and) Capryloyl glycine (and) Water* | 3.0 |
| D | Bioxan T70 | *Tocopherol* | 0,1 |
| E | 50%ige wässrige Zitronensäure | *Citric acid (and) Aqua* | q.s. |

Xanthan Gummi wird in Wasser dispergiert und die wässrige Mischung wird auf 75-80°C erhitzt (Phase A). Die Inhaltsstoffe der Phase B werden unter Erwärmen auf 80 °C miteinander vermischt. Phase B wird unter Rühren bei 75-80°C zur Phase A gegeben. Die Mischung wird 3 min mittels Ultra Turrax dispergiert und anschließend unter Rühren auf < 40 °C abgekühlt. Die erfindungsgemäße Lösung 4E (Phase C) wird unter Rühren zugegeben, gefolgt von Tocopherol (Phase D). Abschließend wird der pH-Wert nach Bedarf auf 5,5 eingestellt. Zur Beurteilung der konservierenden Wirkung der erfindungsgemäßen Lösung wurde das Produkt mit und ohne die erfindungsgemäße Lösung 4E jeweils einem Konservierungsmittelbelastungstest unterzogen:

| | **Creme nach Beispiel 7, mit 3 % der Lösung 4E konserviert (Δ log KBE)** | | | **Creme nach Beispiel 7, jedoch ohne Lösung 4E (Δ log KBE)** | | | **ISO 11930, Kriterien A für ausreichende Konservierung (Δ log KBE)** | | |
|---|---|---|---|---|---|---|---|---|---|
| **Testkeim** | **T7** | **T14** | **T28** | **T7** | **T14** | **T28** | **T7** | **T14** | **T28** |
| **Aspergillus brasiliensis** | - | 0,6 | 0,7 | - | 0,9 | 0,7 | - | ≥ 0 | ≥ 1 |
| **Candida albicans** | 2,1 | 2,7 | 3,5 | -2,1 | -2,1 | -2,1 | ≥ 1 | ≥ 1 | ≥ 1 |
| **Pseudomonas aeruginosa** | ≥ 46 | ≥ 4,6 | ≥ 4,6 | -0,9 | -0,9 | -0,9 | ≥ 3 | ≥ 3 | ≥ 3 |
| **Staphylococcus areus** | ≥ 4,9 | ≥ 4,9 | >4,9 | -0,4 | -0,6 | -0,6 | ≥ 3 | ≥ 3 | ≥ 3 |
| **Escherichia coli** | ≥ 4,7 | ≥ 4,7 | ≥ 4,7 | -0,8 | -0,8 | -0,8 | ≥ 3 | ≥ 3 | ≥ 3 |

Die mit 3 % der erfindungsgemäßen Lösung nach Beispiel 4E konservierte Creme erfüllt die A-Kriterien für ausreichende Konservierung kosmetischer Produkte gemäß ISO 11930, während die unkonservierte Creme die Kriterien der Norm für 4 der 5 Keime nicht erfüllt (Toleranz: ± 0,5 log KBE).

### Beispiel 8 - Konservierung eines Duschgels

| **Phase** | **Inhaltstoff** | **INCI-Name** | **%** |
|---|---|---|---|
| A | Wasser | *Aqua* | 72.4 |
| | Xanthan-Gummi | *Xanthan gum* | 0.6 |
| | Coco-Glucosid | *Cocoglucoside* | 15.0 |
| | Plantapon ACG HC | *Sodium cocoamphoacetate* | 5.0 |
| | Tegobetain F50 | *Cocamidopropyl betain* | 5.0 |
| B | **Erfindungsgemäße Lösung gemäß Beispiel 4C** | *Pentylene glycol (and) Water (and) Sodium benzoate (and) Benzoic acid* | 2.0 |
| C | Zitronensäure (50 % wässr. Lsg.) | *Citric acid (and) Aqua* | qs |

Xanthan-Gummi und Wasser werden bei 700-800 U/min bis zur vollständigen Hydratisierung des Geliermittels gerührt. Die übrigen Komponenten der Phase A werden bei 400 U/min in der angegebenen Reihenfolge zugegeben. Die erfindungsgemäße Lösung wird unter Rühren zugegeben. Anschließend wird der pH-Wert nach Bedarf auf 5,5 abgesenkt (qs = quantum satis, nach Bedarf).

Zur Beurteilung der konservierenden Wirkung der erfindungsgemäßen Lösung wurde das Produkt mit und ohne die erfindungsgemäße Lösung 4C jeweils einem Konservierungsmittelbelastungstest unterzogen:

| **Testkeim** | **Gel nach Beispiel 8, mit 2 % der Lösung 4C konserviert (Δ log KBE)** | | | **Gel nach Beispiel 8, jedoch ohne Lösung 4C (Δ log KBE)** | | | **ISO 11930, Kriterien A für ausreichende Konservierung (Δ log KBE)** | | |
|---|---|---|---|---|---|---|---|---|---|
| | **T7** | **T14** | **T28** | **T7** | **T14** | **T28** | **T7** | **T14** | **T28** |
| **Aspergillus brasiliensis** | >2,6 | >2,6 | >2,6 | -0,2 | -0,1 | -1,0 | - | ≥ 0 | ≥ 1 |
| **Candida albicans** | 2,1 | >3,3 | >3,3 | >3,3 | >3,3 | >3,3 | ≥ 1 | ≥ 1 | ≥ 1 |
| **Pseudomonas aeruginosa** | >4,8 | >4,8 | >4,8 | 2,3 | >4,8 | >4,8 | ≥ 3 | ≥ 3 | ≥ 3 |
| **Staphylococcus areus** | >4,5 | >4,5 | >4,5 | -0,7 | 2,2 | -0,8 | ≥ 3 | ≥ 3 | ≥ 3 |
| **Escherichia coli** | >4,7 | >4,7 | >4,7 | 0,8 | 1,2 | >4,7 | ≥ 3 | ≥ 3 | ≥ 3 |

Das mit 2 % der erfindungsgemäßen Lösung nach Beispiel 4C konservierte Duschgel erfüllt die A-Kriterien für ausreichende Konservierung kosmetischer Produkte gemäß ISO 11930, während das unkonservierte Gel die Kriterien der Norm für 4 der 5 Testkeime nicht erfüllt.

### Beispiel 9 - Konservierung eines Duschgels

| **Phase** | **Inhaltstoff** | **INCI-Name** | % |
|---|---|---|---|
| A | Wasser | *Aqua* | 71.9 |
| | Xanthan-Gummi | *Xanthan gum* | 0.6 |
| | Coco-Glucosid | *Cocoglucoside* | 15.0 |
| | Plantapon ACG HC | *Sodium cocoamphoacetate* | 5.0 |
| | Tegobetain F50 | *Cocamidopropyl betain* | 5.0 |
| B | **Erfindungsgemäße Lösung gemäß Beispiel 4H** | *Pentylene glycol (and) Sodium salicylate (and) Water (and) Salicylic acid* | 2,5 |
| C | Zitronensäure (50 % wässr. Lsg.) | *Citric acid (and) Aqua* | qs |

Die Zubereitung erfolgt analog zu Beispiel 8. Das Duschgel wurde mit und ohne die erfindungsgemäße Lösung 4H jeweils einem Konservierungsmittelbelastungstest unterzogen:

| **Testkeim** | **Gel nach Beispiel 9, mit 2,5 % der Lösung 4H konserviert (Δ log KBE)** | | | **Gel nach Beispiel 9, jedoch ohne Lösung 4H (Δ log KBE)** | | | **ISO 11930, Kriterien A für ausreichende Konservierung (Δ log KBE)** | | |
|---|---|---|---|---|---|---|---|---|---|
| | **T7** | **T14** | **T28** | **T7** | **T14** | **T28** | **T7** | **T14** | **T28** |
| **Aspergillus brasiliensis** | 3,4 | > 5 | > 5 | -0,2 | -0,1 | -1,0 | - | ≥ 0 | ≥ 1 |
| **Candida albicans** | 2,1 | 2,3 | >6 | >3,3 | >3,3 | >3,3 | ≥ 1 | ≥ 1 | ≥ 1 |
| **Pseudomonas aeruginosa** | > 6 | > 6 | > 6 | 2,3 | >4,8 | >4,8 | ≥ 3 | ≥ 3 | ≥ 3 |
| **Staphylococcus areus** | > 6 | > 6 | > 6 | -0,7 | 2,2 | -0,8 | ≥ 3 | ≥ 3 | ≥ 3 |
| **Escherichia coli** | > 6 | > 6 | > 6 | 0,8 | 1,2 | >4,7 | ≥ 3 | ≥ 3 | ≥ 3 |

Das mit 2,5 % der erfindungsgemäßen Lösung nach Beispiel 4H konservierte Duschgel erfüllt die A-Kriterien für ausreichende Konservierung kosmetischer Produkte gemäß ISO 11930, während das unkonservierte Gel die Kriterien der Norm für 4 der 5 Testkeime nicht erfüllt.

## Patentansprüche

1. Stabile homogene Lösung,
enthaltend folgende Komponenten:
a) zu 40 - 95 Gew.-% mindestens eines C₅-C₆-Alkandiols,
b) zu 0,1 - 35 Gew.-% mindestens einer Carbonsäure aus der Gruppe Benzoesäure, Salicylsäure und Capryloylglycin,
c) zu 0,1 - 35 Gew.-% mindestens eines Salzes einer C₆-C₉-Carbonsäure,
d) zu 0,1 - 25 Gew.-% Wasser,
wobei sich die Anteile a, b, c und d zu 100 Gew.-% addieren.

2. Lösung gemäß Anspruch 1, **dadurch gekennzeichnet, dass** das mindestens eine Alkandiol ein vicinales C₅-C₆-Alkandiol ist.

3. Lösung nach mindestens einem der Ansprüche 1 und 2, **dadurch gekennzeichnet, dass** das Alkandiol 1,2-Pentandiol oder 1,2-Hexandiol ist.

4. Lösungen nach mindestens einem der Ansprüche 1 bis 3, **dadurch gekennzeichnet, dass** die mindestens eine Carbonsäure Benzoesäure ist.

5. Lösungen nach mindestens einem der Ansprüche 1 bis 3, **dadurch gekennzeichnet, dass** die mindestens eine Carbonsäure Salicylsäure ist.

6. Lösung nach mindestens einem der Ansprüche 1 bis 5, **dadurch gekennzeichnet, dass** das mindestens eine Salz einer Carbonsäure ein Carboxylat einer Carbonsäure aus der Gruppe Benzoesäure und Salicylsäure ist.

7. Lösung nach mindestens einem der Ansprüche 1 bis 6, **dadurch gekennzeichnet, dass** das Gegenion des Carboxylats der Carbonsäure ausgewählt ist unter Ammonium-, Triethanolammonium-, Natrium-, Kalium-, Calcium- und Magnesium-Kation.

8. Verwendung einer Lösung nach mindestens einem der Ansprüche 1 bis 7 zur Konservierung kosmetischer und dermatologischer Produkte.

9. Verwendung einer Lösung nach mindestens einem der Ansprüche 1 bis 7 zur Hemmung des Wachstums und Abtötung von Mikroorganismen.

10. Verfahren zur Wachstumshemmung oder Abtötung von Mikroorganismen, **dadurch gekennzeichnet, dass** eine Lösung nach mindestens einem der Ansprüche 1 bis 7 einem Produkt aus den Bereichen Kosmetik, Dermatologie, Körperpflege und Körperhygiene zugesetzt wird.

## Claims

1. Stable homogeneous solution,
containing the following components:
a) 40-95% by weight of at least one C₅-C₆ alkanediol,
b) 0.1-35% by weight of at least one carboxylic acid from the group of benzoic acid, salicylic acid and capryloylglycine,
c) 0.1-35% by weight of at least one salt of a C₆-C₉ carboxylic acid,
d) 0.1-25% by weight of water,
wherein the proportions a, b, c and d add up to 100% by weight.

2. Solution according to claim 1, **characterized in that** the at least one alkanediol is a vicinal C₅-C₆ alkanediol.

3. Solution according to at least one of claims 1 and 2, **characterized in that** the alkanediol is 1,2-pentanediol or 1,2-hexanediol.

4. Solutions according to at least one of claims 1 to 3, **characterized in that** the at least one carboxylic acid is benzoic acid.

5. Solutions according to at least one of claims 1 to 3, **characterized in that** the at least one carboxylic acid is salicylic acid.

6. Solution according to at least one of claims 1 to 5, **characterized in that** the at least one salt of a carboxylic acid is a carboxylate of a carboxylic acid from the group of benzoic acid and salicylic acid.

7. Solution according to at least one of claims 1 to 6, **characterized in that** the counterion of the carboxylate of the carboxylic acid is selected among ammonium, triethanolammonium, sodium, potassium, calcium and magnesium cation.

8. Use of a solution according to at least one of claims 1 to 7 for the preservation of cosmetic and dermatological products.

9. Use of a solution according to at least one of claims 1 to 7 for inhibiting the growth and killing of microorganisms.

10. Method for inhibiting the growth or killing of microorganisms, **characterized in that** a solution according to at least one of claims 1 to 7 is added to a product from the fields of cosmetics, dermatology, personal care and personal hygiene.

## Revendications

1. Solution homogène stable,
contenant les composants suivants:
a) 40 à 95% en poids d'au moins un alcanediol en C₅-C₆,
b) 0,1 à 35% en poids d'au moins un acide carboxylique du groupe constitué par l'acide benzoïque, l'acide salicylique et la capryloylglycine,
c) 0,1 à 35% en poids d'au moins un sel d'un acide carboxylique en C₆-C₉,
d) 0,1 à 25% en poids d'eau,
où les proportions a, b, c et d totalisent 100% en poids.

2. Solution selon la revendication 1, **caractérisée en ce que** l'au moins un alcanediol est un alcanediol en C₅-C₆ vicinal.

3. Solution selon au moins l'une quelconque des revendications 1 et 2, **caractérisée en ce que** l'alcanediol est le 1,2-pentanediol ou le 1,2-hexanediol.

4. Solutions selon au moins l'une quelconque des revendications 1 à 3, **caractérisées en ce que** le au moins un acide carboxylique est l'acide benzoïque.

5. Solutions selon au moins l'une quelconque des revendications 1 à 3, **caractérisées en ce que** le au moins un acide carboxylique est l'acide salicylique.

6. Solution selon au moins l'une quelconque des revendications 1 à 5, **caractérisée en ce que** le au moins un sel d'un acide carboxylique est un carboxylate d'un acide carboxylique du groupe de l'acide benzoïque et de l'acide salicylique.

7. Solution selon au moins l'une quelconque des revendications 1 à 6, **caractérisée en ce que** le contre-ion du carboxylate de l'acide carboxylique est choisi parmi les cations ammonium, triéthanolammonium, sodium, potassium, calcium et magnésium.

8. Utilisation d'une solution selon au moins l'une quelconque des revendications 1 à 7 pour la conservation de produits cosmétiques et dermatologiques.

9. Utilisation d'une solution selon au moins l'une quelconque des revendications 1 à 7 pour inhiber la croissance et la destruction des micro-organismes.

10. Procédé pour inhiber la croissance ou la destruction de micro-organismes, **caractérisé en ce qu'**une solution selon au moins l'une quelconque des revendications 1 à 7 est ajoutée à un produit des domaines de la cosmétique, de la dermatologie, des soins personnels et de l'hygiène personnelle.
